# FASCICULE DE BREVET EUROPEEN

(11) **EP 3 520 619 B1**
(45) Date de publication et mention de la délivrance du brevet: **06.01.2021**
(21) Numéro de dépôt: 19155091.2
(22) Date de dépôt: 01.02.2019
(51) Int. Cl.: A21D 8/04, A21D 4/00, B65D 81/20

(54) **LEVAIN ET SON PROCEDE DE CONDITIONNEMENT**
HEFETEIG UND SEIN VERPACKUNGSVERFAHREN
LEAVENING AGENT AND METHOD FOR PACKAGING SAME

(30) Priorité: 02.02.2018 FR 1870117
(43) Date de publication de la demande: 07.08.2019
(73) Titulaire: Philibert, Pascal, 01750 Replonges (FR)
(72) Inventeur: PHILIBERT, Pascal, 01750 Replonges (FR); VERA, Annabelle, 69290 Craponne (FR)
(74) Mandataire: Weber, Jean-François

(56) Documents cités:
- EP-A1- 0 636 692
- WO-A1-02/02409
- WO-A1-2009/136248
- CN-A- 101 531 985
- FR-A1- 3 040 587
- RU-C1- 2 146 091

## Description

La présente invention se rapporte au conditionnement de levains ou assimilés pour garantir de bonnes conditions de conservation et rallonger la durée de vie du produit.

On entend par "levains ou assimilés" tout produit fermenté à partir de céréales (blé tendre, blé dur, seigle par exemple), de pseudo-céréales (sarrasin, quinoa par exemple), ou de légumineuse, et sous toutes leurs formes (graines entières sèches, graines germées, farines, mélange de farines, semoules et toutes autres fractions (les sons, germes par exemple).

Le levain est un produit fermenté ancestral, utilisé depuis l'Antiquité, pour fabriquer du pain et toutes sortes de pâtes levées, qui renferme une microflore vivante constituée majoritairement de bactéries lactiques et de levures. Il peut être obtenu par fermentation spontanée des microorganismes naturellement présents dans le substrat de fermentation, généralement de la farine, ou à partir d'un ensemencement avec des microorganismes sélectionnés appelés starters. Le document RU 2 146 091 par exemple, décrit l'emballage d'un levain comprenant de 43 à 46% d'humidité dans un film imperméable à l'eau, puis sa conservation par congélation entre -18 et -24°C.

L'activité fermentaire des microorganismes du levain, plus particulièrement des bactéries lactiques, durant la panification est essentielle et confère au pain fabriqué avec du levain toute sa richesse aromatique et ses notes acidulées typiques. Les microorganismes métaboliquement actifs libèrent des acides organiques, de nombreux composés aromatiques et précurseurs d'arômes, mais également d'autres composés d'intérêt comme des exopolysaccharides, des antifongiques et entament une dégradation des constituants de la pâte durant la panification. Ainsi, le levain vivant affecte l'ensemble des attributs organoleptiques du pain (texture de la mie plus grasse, alvéolage irrégulier, une croute plus épaisse et plus croustillante), améliore sa conservation, en ralentissant le rassissement et le développement des moisissures, et modifie la digestibilité du pain, en favorisant notamment la biodisponibilité des minéraux et en réduisant son index glycémique.

Le levain vivant se différencie du levain dévitalisé en premier lieu sur sa composition microbiologique, à savoir que le levain dévitalisé ne contient plus de microorganismes vivants et a perdu son activité fermentaire. Le levain vivant est généralement stabilisé par le froid, réfrigéré et a une durée de conservation plus limitée dans le temps, alors que le levain dévitalisé, dont la microflore a été détruite par des procédés divers, comme la déshydratation à haute température, la pasteurisation ou encore par pression osmotique, se conserve généralement longtemps à température ambiante non contrôlée. Toutefois, le levain dévitalisé ne présente plus d'activité fermentaire et est utilisé uniquement pour une fonction d'aromatisation de la pâte.

L'invention concerne tous les types de levains ou assimilés qui ont un pourcentage de matière sèche (MS) en poids inférieur à 90%, notamment inférieur à 75% ou à 70% et qui sont actifs/vivants, qui peuvent subir des altérations liées à l'oxygène de leurs propriétés organoleptiques, nutritionnelles et microbiennes au cours de leur conservation en conditions de froid positif, de froid négatif ou à température ambiante non contrôlée. Il s'agit de phénomènes d'oxydation, de brunissement enzymatique ou non enzymatique, de dégradations enzymatiques ou encore microbiennes.

Ces altérations liées à l'oxygène de l'air conduisent à la diminution de la qualité organoleptique (évolution de la couleur ou des arômes par exemple), de la qualité nutritionnelle (oxydation des acides gras insaturés, des vitamines par exemple) et de l'évolution microbienne du levain ou assimilé (développement de flores aérobies indésirables, par exemple de moisissures, ou diminution des flores technologiques, notamment les bactéries lactiques anaérobies).

WO 02/02409 (Air Liquide) concerne le conditionnement de produits périssables, notamment sensibles au développement de microorganismes en vue de prolonger leur durée de conservation en assurant leur sécurité microbiologique. Il est également précisé :
Par "sécurité microbiologique", on entend des conditions de conservation permettant d'éviter, ou pour le moins de réduire à un taux acceptable, c'est-à-dire non-toxique pour le consommateur et satisfaisant à la réglementation en vigueur pour les produits considérés, le développement de microorganismes.

Le terme "microorganismes" englobe les microorganismes pathogènes, mais également des microorganismes non pathogènes pouvant altérer les qualités et/ou l'activité du produit, notamment ceux pouvant engendrer un aspect ou une odeur désagréable ou ceux pouvant modifier ou dégrader les propriétés nutritionnelles, chimiques et/ou pharmaceutiques des produits.

WO 02/02409 (Air Liquide) revendique le conditionnement avec un gaz protecteur formé d'hydrogène et éventuellement de gaz de conditionnement, qui peuvent être des gaz inertes comme l'azote ou l'argon, ou encore des gaz pouvant avoir une interaction protectrice tels que le dioxyde de carbone ou protoxyde d'azote.

L'objectif d'Air Liquide est d'éviter et de réduire le développement des microorganismes des denrées alimentaires. Or, un levain est un produit vivant dont on souhaite conserver l'activité des microorganismes caractéristiques qui le composent, les bactéries lactiques et les levures. Par conservation des microorganismes du levain, on entend garder les microorganismes dans un état viable (vivants) et cultivable, c'est-à-dire qu'ils soient capables de reprendre une activité métabolique en conditions plus favorables. Le levain est un produit naturellement stable vis-à-vis des flores indésirables, car c'est un produit acide.

Les essais de l'exemple 3 de conservation du levain à la base de la présente invention ont montré que seule l'atmosphère protectrice sensiblement 100% azote N2, qui est un gaz inerte, a permis de stabiliser le taux de bactéries lactiques du levain. L'atmosphère contenant de l'hydrogène, notamment 4.5% H2/95.5% N2 s'est révélée délétère à partir de 6 semaines de conservation, tout comme l'air. Au niveau du taux de bactéries lactiques, on considère une différence "réelle" pour un écart > 0.5 log(ufc)/g. Ces essais ont montré que l'écart entre le conditionnement sous 100% N2 et celui sous 4.5% H2/95.5% N2 était de 1.77 et 1.68 log(ufc)g à respectivement 6 et 8 semaines de conservation du levain, soit 47.75 fois plus de bactéries vivantes présentes à 8 semaines de conservation dans le levain sous N2. L'invention permet aussi de conserver (stabiliser) la teneur en acides gras d'un levain, notamment en acides gras insaturés.

L'invention a pour objet un produit consistant en du levain ou assimilé ayant un pourcentage de matière sèche en poids inférieur à 90%, vivant, caractérisé en ce que le produit est contenu, sous une atmosphère d'azote à une pureté supérieure à 97%, de préférence comprise entre 99 et 100%, en volume dans un emballage en une matière barrière au gaz et fermé de manière étanche au gaz.

Le levain ou assimilé est conditionné de manière à limiter le contact avec l'oxygène. Le levain est conditionné de manière à avoir un taux d'O₂ résiduel dans l'atmosphère à l'intérieur de l'emballage <5%, et de préférence <2%. Pour cela, le levain est de préférence conditionné sous atmosphère modifiée protectrice dans un emballage dont le matériau fait barrière aux gaz. L'emballage est fermé de manière étanche aux gaz.

L'atmosphère protectrice doit être non oxydante. On remplace l'air ambiant dans un récipient d'emballage par de l'azote. L'azote (N₂) peut avoir une pureté >97%, notamment de 99 à 100% en volume.

Le matériau du récipient d'emballage peut-être de n'importe quelle nature (composition), souple ou rigide, et doit faire hautement barrière aux gaz, principalement à l'oxygène (O₂), au dioxyde de carbone (CO₂), à l'azote (N₂) et à la vapeur d'eau. Plus particulièrement pour l'oxygène, le matériau du récipient d'emballage doit avoir un coefficient de perméabilité à l'O₂ <30 cm³/m²/24h.bar à 23°C, de préférence <10 cm³/m²/24h.bar à 23°C. Le matériau est de préférence une structure multicouche hautement barrière aux gaz. Il peut être par exemple composé entre autres de PVDC (polychlorure de vinylidène), OPA (polyamide orienté), EVOH (polyéthylène alcool vinylique), d'aluminium, de silice type SiOx et/ou de verre ou autres. La matière d'emballage peut de préférence être anti-UV.

L'emballage peut se présenter sous n'importe quelle forme (barquette operculée ou sachet par exemple).

Le conditionnement peut être fait par n'importe qu'elle machine permettant d'obtenir un emballage sous vide ou sous atmosphère protectrice, préférentiellement par des techniques de balayage gazeux ou par vide compensé et injection de gaz. L'espace occupé par le gaz (appelé espace de tête) doit être >10% du volume total de l'emballage et cette atmosphère doit avoir un taux résiduel d'O₂ <5%.

L'invention a aussi pour objet un procédé dans lequel on met du levain ou assimilé, vivant, dans un récipient en une matière barrière aux gaz sous atmosphère d'azote pur à plus de 97% en volume et on ferme le récipient de manière étanche au gaz.

On utilise les méthodes d'analyse suivantes :
Méthode d'analyse composition gazeuse : La composition gazeuse en O2 et CO2 de l'espace de tête est mesurée à l'aide d'un analyseur O2/CO2 modèle 30 Oxybaby (marque de fabrique) 6.0 (WITT). Pour chaque point d'analyse, la méthode est destructive (piqûre à travers l'emballage et analyse du gaz dans l'espace de tête du sachet). Le sachet est ensuite ouvert pour les analyses suivantes. Cet analyseur mesure par capteur directement les taux d'O2 et de CO2 puis calcule par différence le pourcentage de "gaz autres" que l'O2 et CO2 contenus dans l'atmosphère gazeuse, la somme de tous les gaz étant égale à 100%. Dans les essais de l'exemple, les gaz autres sont composés de N2 principalement pour l'exemple 3A, de N2 exclusivement pour l'exemple 3B et de H2+N2 pour l'exemple 3C.

Méthode d'analyse des bactéries lactiques : Le taux de bactéries lactiques a été déterminé selon la norme de référence NF ISO 15214 (indice de classement V 08-030), méthode de référence, de la manière suivante : L'ensemencement se fait en surface sur gélose MRS (Biokar (marque de fabrique)) préalablement coulée dans des boîtes de pétri avec une quantité déterminée de suspension mère et/ou des dilutions décimales de l'échantillon. Le dénombrement s'effectue après incubation des boîtes en anaérobiose à 30°C pendant 72 à 120 heures. Le résultat est exprimé en logarithme décimal d'unités formant colonies par gramme de levain (log(ufc)/g).

Méthode d'analyse des acides gras : la teneur en matières grasses totales ainsi que la composition en acides gras sont mesurées respectivement par gravimétrie (Soxhlet) et par technique GC/FID. La matière grasse est hydrolysée et extraite avec de l'acide sulfurique et du cyclohexane par techniques micro-ondes. La matière grasse est déterminée par gravimétrie après évaporation du cyclohexane.

Pour profil des acides gras, la matière grasse est extraite, puis préparée par méthylation et analysée par chromatographie en phase gazeuse (CPG) avec étalonnage direct. Les résultats sont exprimés en pourcentage de la somme des acides gras totaux composant le levain.

Seuls les acides gras correspondant à plus de 0,05% des acides gras totaux sont quantifiés.

La matière sèche (MS) est mesurée par méthode infrarouge avec un dessiccateur halogène Radwag (marque de fabrique) modèle MAC 50/1. Les mesures sont réalisées en triple sur 2,0 ± 0,1 g de produit, puis moyennées. Le programme d'analyse applique une température de 130°C jusqu'à ce que la variation de la valeur de pesée soit ≤ 1 mg en 25 s. Les valeurs sont exprimées en pourcentage de MS contenue dans le levain ou assimilé.

La couleur du levain est suivie par analyse instrumentale à l'aide d'un colorimètre MINOLTA CR410 selon l'espace chromatique L*a*b*, dont les coordonnées colorimétriques mesurées sont :
le "L*" : niveau de noir/blanc (échelle de 0 = noir pur à 100 = blanc pur) ;
- le "a*" : niveau de vert/rouge (échelle de -60 = vert pur à +60 = rouge pur) ;
- le "b*" : niveau de bleu/jaune (échelle de -60 = bleu absolu à +60 = jaune absolu).

### Exemple n°1 suivant l'invention :

On conditionne 500 g de levain de germe de blé dont la MS est de 32,5% (levain frais vivant) dans un sachet en PET enduit PVDC-PE dont le coefficient de perméabilité à l'O₂ est <10 cm³/m²/24h.bar, sous une atmosphère protectrice composée de 100% azote (MESSER (marque de fabrique)) pour usage alimentaire conforme au règlement CE n° 1333/2008. Le conditionnement est réalisé à l'aide d'une cloche à vide compensé CCM (marque de fabrique) série BORA 550 Dual Gas selon les paramètres suivants : 8 secondes de tirage au vide, 8 secondes d'injection du gaz à une pression de 3 bars, 5 secondes de thermoscellage. Le volume occupé par l'atmosphère protectrice (espace de tête) représente 40% du volume total dans le sachet.

Le levain emballé est ensuite conservé en chambre froide classique à une température de 4-6°C dans une atmosphère d'air normale (composée d'environ 20 à 21% d'O₂).

### Exemple n°2 : (comparatif)

On conditionne 5kg de levain de germe de blé dont la MS est <de 32,5%% (levain frais vivant) dans un seau en PP (polypropylène) dont le coefficient de perméabilité à est de 40 cm³/m²/24h.bar. Le conditionnement est fait en conditions d'air normales, aucune atmosphère protectrice n'est appliquée, ni de mise sous vide. Le volume d'espace de tête dans le seau est de 20% du volume total du seau. Le levain dans le seau est ensuite conservé en chambre froide classique à une température de 4-6°C dans une atmosphère d'air normale (composée d'environ 20 à 21% d'O2).

La matière sèche (MS) est mesurée par méthode infrarouge avec un dessiccateur halogène Radwag (marque de fabrique) modèle MAC 50/1. Les mesures sont réalisées en triple sur 2,0 + 0,1 g de produit, puis moyennées. Le programme d'analyse applique une température de 130°C jusqu'à ce que la variation de la valeur de pesée soit ≤ 1mg en 25s. Les valeurs sont exprimées en pourcentage de MS contenue dans le levain ou assimilé.

La composition gazeuse en O₂ et CO₂ de l'espace de tête est mesurée à l'aide d'un analyseur O₂/CO₂ modèle Oxybaby (marque de fabrique) 6.0 (WITT). Pour chaque point de mesure, deux sachets sont analysés par méthode destructive (piqûre à travers l'emballage et analyse du gaz dans l'espace de tête du sachet).

Pour l'exemple 1 : la mesure de couleur est effectuée sur le levain préalablement homogénéisé dans le sachet (par pression manuelle du sachet).

Pour l'exemple 2 : on effectue un prélèvement du levain contenu dans le seau en PP. On prélève 50g de la couche supérieure du levain en contact avec l'espace de tête (2 cm environ de hauteur) et 50g de la couche inférieure puis on les mélange. La mesure de la couleur est réalisée sur ce mélange.

### Résultats dans le tableau 1

| | | Exemple 1 | Exemple 2 |
|---|---|---|---|
| taux d'O₂ résiduel dans l'espace de tête (en %) | 3 jours | 1,2 | - |
| | 0,5 mois | 0,1 | - |
| | 1 mois | 0 | - |
| | 1,5 mois | 0,4 | - |
| couleur axe L* | 3 jours | 67,16 | 65,59 |
| | 0,5 mois | 67,93 | 43,46 |
| | 1 mois | 68,08 | 46,44 |
| | 1,5 mois | 68,11 | 44,91 |
| couleur axe a* | 3 jours | 4,12 | 2,72 |
| | 0,5 mois | 4,08 | -0,88 |
| | 1 mois | 4,36 | -1,23 |
| | 1,5 mois | 4,32 | -0,84 |
| couleur axe b* | 3 jours | 20,83 | 19,01 |
| | 0,5 mois | 20,32 | -2,12 |
| | 1 mois | 20,67 | -0,5 |
| | 1,5 mois | 20,92 | -0,87 |

Les résultats obtenus montrent que dans l'exemple 1 le taux d'oxygène résiduel à l'intérieur de l'emballage est <2%, comme préconisé dans l'invention, et que cela permet de maintenir une couleur stable au long de la conservation du levain. Peu de variations sur les paramètres colorimétriques L*a*b*, le levain a gardé sa couleur d'origine marron clair.

Par contre dans l'exemple 2 l'atmosphère dans l'emballage n'a pas été modifiée, l'espace de tête était donc composé d'air normal lors du conditionnement (l'air étant composé de 20-21% d'02) et le seau en PP n'était pas suffisamment barrière à l'oxygène. Cela s'est traduit par une évolution importante de la couleur (coloration brun/noir) du levain au cours de la conservation), principalement sur le paramètre colorimétrique L* dont la mesure passe de 65.59 (levain assez clair) à 44,91 après 1.5 mois de conservation (levain très foncé gris/noir). Le levain a également évolué sur les axes a* et b* vers le vert et le bleu.

### Exemples 3A et 3C (comparatifs) et 3B suivant l'invention :

### Essais menés sous air ou sous hydrogène par rapport à l'azote :

On conditionne 500 g de levain de germe de blé dont la MS est de 32,5% en poids (levain frais vivant) dans des sachets en OPP/PE-EVOH-PE dont le coefficient de perméabilité à l'O2 est égal à 2 cm3/m2/24h.bar, sous une atmosphère non modifiée composée d'air (exemple 3A) ou sous une atmosphère modifiée composée de 100% azote (MESSER (marque de fabrique) (exemple 3B) ou composée de 4.5% H2/95.5% N2 (LINDE (marque de fabrique) (exemple 3C). Le conditionnement est réalisé à l'aide d'une cloche à vide compensé CCM (marque de fabrique) série BORA 550 Dual Gas selon les paramètres suivants : 8 secondes de tirage au vide, 8 secondes d'injection du gaz à une pression de 3 bar, 3 ou 4 secondes de thermoscellage.

Le volume occupé par l'atmosphère protectrice (espace de tête) représente 40 à 50% du volume total dans le sachet.

Le levain conditionné est ensuite conservé en chambre froide classique à une température de 4-6°C dans une atmosphère d'air normale (composée d'environ 20 à 21% d'02) .

On réalise un suivi du levain durant 8 semaines, notamment de la composition gazeuse de l'espace de tête des sachets, de la couleur du levain, de sa composition en bactéries lactiques et de sa composition en acides gras.

### Evolution de la composition gazeuse

| | **Temps de conservation en semaines** | **Taux d'O2 en %** | **Taux de CO2 en %** | **Autres gaz = taux de N2 ou N2+H2** |
|---|---|---|---|---|
| sous air (3A) | 0 | 21,13 | 0,4 | 78,5 |
| | 0,5 | 19,1 | 5,4 | 75,5 |
| | 1 | 18,4 | 7,3 | 74,3 |
| | 2 | 17 | 8,2 | 74,8 |
| | 3 | 16,1 | 11,1 | 72,8 |
| | 4 | 20,3 | 2,4 | 77,3 |
| | 6 | 9,1 | 13,5 | 77,4 |
| | 8 | 12,5 | 5,2 | 82,3 |
| sous 100% N2 (3B) | 0 | 0,33 | 0,5 | 99,2 |
| | 0,5 | 2,3 | 2,8 | 94,9 |
| | 1 | 0,5 | 7,1 | 92,4 |
| | 2 | 1,4 | 6,3 | 92,3 |
| | 3 | 0,4 | 13,3 | 86,3 |
| | 4 | 1 | 9,7 | 89,3 |
| | 6 | 0,6 | 9,6 | 89,8 |
| | 8 | 0,6 | 7,4 | 92 |
| sous 4,5% H2 + 95,5% N2 (3C) | 0 | 0,2 | 0,2 | 99,6 |
| | 0,5 | 0 | 4,2 | 95,8 |
| | 1 | 1,4 | 7,9 | 90,7 |
| | 2 | 0,1 | 5,2 | 94,7 |
| | 3 | 0 | 9,2 | 90,8 |
| | 4 | 0,5 | 10,6 | 88,9 |
| | 6 | 1,2 | 10,4 | 88,4 |
| | 8 | 0,5 | 7,4 | 92,1 |

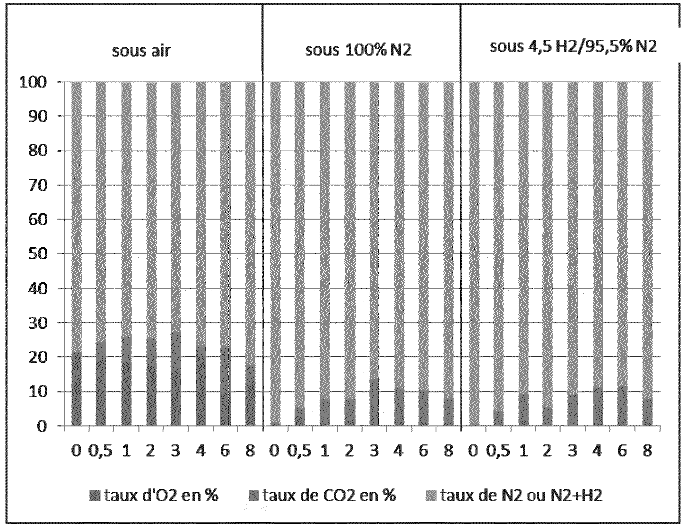

Les résultats obtenus montrent que les taux d'oxygène résiduel à l'intérieur des sachets sous atmosphère modifiée protectrice 100%N2 ou 4.5% H2/95.5% N2 respectent les préconisations de l'invention. Ils sont <5% d'O2 résiduel et majoritairement <2% d'O2 résiduel. Alors que les sachets conditionnés sous air montrent des taux d'O2 résiduel dans l'espace de tête du sachet >9% et principalement >15% d'O2.

Le levain étant un produit vivant, les microorganismes qui le composent, les bactéries lactiques et les levures, ont leur activité métabolique fortement ralentie mais pas totalement inactivée. Cela peut expliquer la production de CO2 dans les sachets sous atmosphère protectrice due au métabolisme fermentaire de la microflore. Sous air, une partie de l'oxygène a également été consommée par les microorganismes.

### Evolution de la couleur :

| | **Temps de conservation en semaines** | **L*** | **a*** | **b*** |
|---|---|---|---|---|
| sous air | 0,5 | 61,65 | 2,13 | 16,72 |
| | 1 | 57,29 | -0,14 | 11,31 |
| | 2 | 59,76 | 0,88 | 14,52 |
| | 3 | 56,21 | -0,21 | 10,13 |
| | 4 | 56,45 | -0,27 | 10,42 |
| | 6 | 56,73 | 0,09 | 11,28 |
| | 8 | 54,25 | 0,08 | 9,15 |
| sous 100% N2 | 0,5 | 62,43 | 2,86 | 17,89 |
| | 1 | 62,55 | 3,16 | 18,38 |
| | 2 | 63,85 | 3,86 | 19,72 |
| | 3 | 63,29 | 3,61 | 18,89 |
| | 4 | 63,24 | 3,42 | 18,75 |
| | 6 | 62,58 | 3,39 | 18,74 |
| | 8 | 62,78 | 3,89 | 18,86 |
| sous 4,5% H2 + 95,5% N2 | 0,5 | 63,4 | 4,22 | 20,77 |
| | 1 | 63,63 | 4,04 | 19,92 |
| | 2 | 64,23 | 4,22 | 20,25 |
| | 3 | 64,03 | 4,19 | 19,85 |
| | 4 | 64,07 | 4,19 | 20,17 |
| | 6 | 63,96 | 4,26 | 20,22 |
| | 8 | 62,26 | 3,84 | 18,73 |

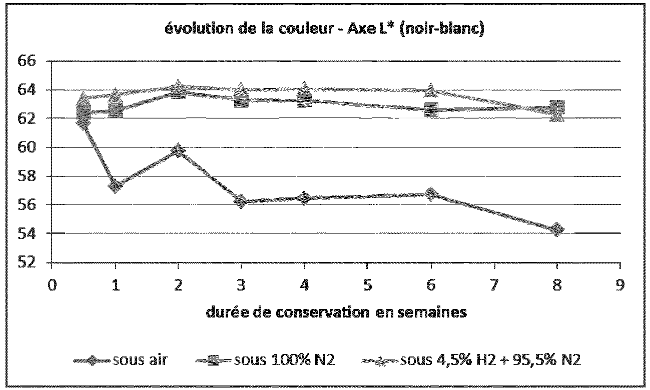

Les atmosphères modifiées 100% N2 et 4.5% H2/95.5% N2 ont un effet sur la couleur du levain équivalent. Elles permettent toutes deux de conserver les propriétés de couleur du levain, donc d'empêcher l'oxydation du levain, alors qu'il y a une forte évolution de la couleur sous air. La figure ci-dessus illustre l'effet sur l'axe de couleur L* dans le système L*a*b*, dont la valeur diminue rapidement ce qui se traduit par une coloration du levain qui tend vers le noir de manière importante, ce qui rend le levain impropre à la vente. Alors que sous les atmosphères modifiées 100% N2 et 4.5% H2/95.5% N2 le paramètre L* reste stable, tout comme les autres paramètres a* et b*.

### Evolution de la microflore lactique :

| | taux de bactéries lactiques en log(ufc)/g | | |
|---|---|---|---|
| nombre de semaines | sous air | sous 100% N2 | sous 4,5% H2 + 95,5% N2 |
| 0,5 | 7,11 | 7,65 | 7,58 |
| 1 | 7,38 | 7,48 | 7,62 |
| 2 | 7,32 | 7,00 | 7,30 |
| 3 | 7,52 | 7,40 | 7,30 |
| 4 | **5,30** | **7,18** | **6,78** |
| 6 | **4,04** | **6,85** | **5,08** |
| 8 | **4,85** | **6,96** | **5,28** |

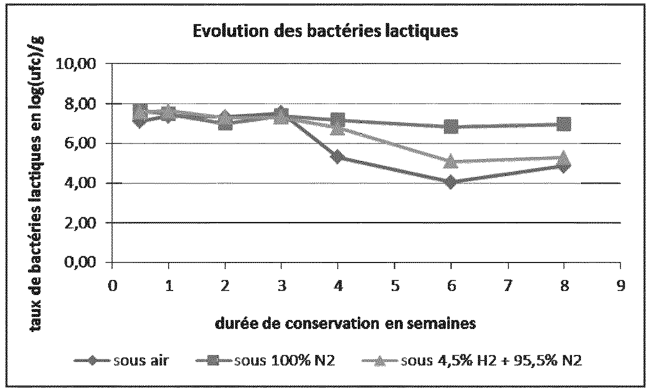

### Evolution de la composition en acides gras :

| | temps de conservatio n en semaines | MG totales g/100g | Profil des acides gras en % | | | différence avec t0 | | |
|---|---|---|---|---|---|---|---|---|
| | | | AG saturés | AG mono-insaturés | AG poly-insaturés | AG *saturés* | *AG* mono *insaturés* | *AG poly insaturés* |
| avant condi | 0 | 3,5 +/-0,5 | 18,9 | 14,8 | 66,3 | - | - | - |
| 100% N2 | | 3,8 +/ - 0,6 | 19,82 | 15,92 | 64,15 | +0,92 | +1,12 | -2,15 |
| H2+N2 | 2 sem | 3,4 +/-0,6 | 25,98 | 14,46 | 59,56 | **+7,08** | **-0,34** | **-6,74** |
| 100% N2 | | 3,5 +/-0,6 | 18,7 | 15,16 | 66,14 | -0,2 | +0,36 | -0,16 |
| H2+N2 | 4 sem | 3,5 +/-0,6 | 23,19 | 12,45 | 64,36 | **+4,29** | **-2,35** | **-1,94** |
| MG : matières grasses | | | | | | | | |
| AG : acides gras | | | | | | | | |

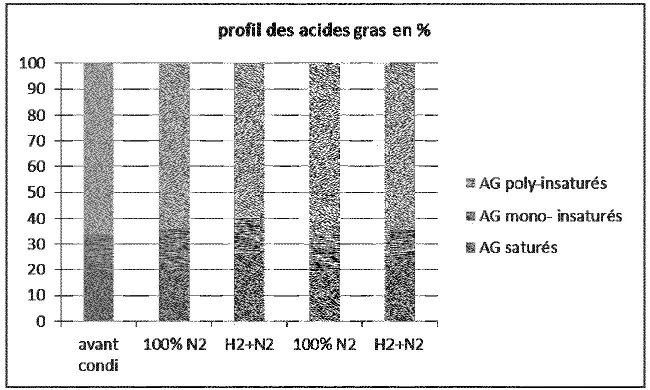

L'atmosphère modifiée 4.5% H2/95.5% N2 a impacté le profil des acides gras du levain en augmentant la composition en acides gras saturés et proportionnellement en diminuant la composition en acide gras insaturés ; alors que le conditionnement sous 100% N2 a permis de complètement stabiliser la composition en acides gras du levain.

Les acides gras saturés sont des lipides qui ne comportent aucune double liaison dans leur structure chimique. Dans l'alimentation, ils se trouvent principalement dans les produits d'origine animale (beurre, charcuterie...). Alors que les acide gras insaturés comportent une ou plusieurs doubles liaisons dans leur structure et se rencontrent principalement dans les poissons gras et les aliments d'origine végétale, comme c'est le cas du levain de germe de cet exemple. Les acides gras insaturés sont à privilégier dans l'alimentation car ils sont reconnus pour leur effet bénéfique sur la santé, notamment pour leur contribution au bon fonctionnement du système cardiovasculaire.

Le conditionnement selon l'invention, sous 100% N2, permet donc de conserver correctement les propriétés nutritionnelles du levain, alors que celui comportant de l'hydrogène a tendance à favoriser la transformation en acides gras saturés.

De manière inattendue, le conditionnement sous azote permet non seulement de conserver les caractéristiques intrinsèques du levain (couleur, physico-chimiques, composition nutritionnelle), mais également de rallonger la durée de vie de sa microflore, notamment de conserver les bactéries lactiques vivantes plus longtemps, par rapport à un conditionnement classique sous air.

## Revendications

1. Produit consistant en du levain ou assimilé, vivant, ayant un pourcentage de matière sèche en poids inférieur à 90%, **caractérisé en ce que** le produit est contenu sous une atmosphère d'azote à une pureté supérieure à 97%, de préférence comprise entre 99 et 100%, en volume dans un emballage en une matière barrière au gaz et fermé de manière étanche au gaz.

2. Produit suivant la revendication 1, **caractérisé en ce que** la matière de l'emballage a un coefficient de perméabilité à l'O₂ inférieur à 10 cm³/m²/24h.

3. Produit suivant l'une des revendications 1 ou 2, **caractérisé en ce que** la matière de l'emballage est une barrière à la vapeur d'eau.

4. Produit suivant l'une des revendications précédentes, **caractérisé en ce que** la matière comprend une ou plusieurs couches en PVDC, en OPA, en EVOH ou en aluminium ou en leurs mélanges.

5. Produit suivant l'une des revendications précédentes, **caractérisé en ce que** la matière comprend du verre, de la silice et/ou de l'aluminium.

6. Produit suivant l'une des revendications précédentes, **caractérisé en ce que** la matière est anti-UV.

7. Procédé de fabrication d'un produit suivant l'une des revendications précédentes, **caractérisé en ce que** l'on met du levain ou assimilé, vivant, dans un récipient en une matière barrière aux gaz sous atmosphère d'azote pur à plus de 97% en volume et on ferme le récipient de manière étanche au gaz.

8. Procédé suivant la revendication 7, **caractérisé en ce que** l'on utilise de l'azote à une pureté comprise entre 99 et 100% en volume.

## Patentansprüche

1. Produkt, das aus lebendem Sauerteig oder Ähnlichem besteht, das einen Prozentsatz an Trockensubstanz kleiner als 90 Gew.-% aufweist, **dadurch gekennzeichnet, dass** das Produkt unter einer Stickstoffatmosphäre mit einer Reinheit größer als 97 Vol.-%, vorzugsweise zwischen 99 und 100 Vol.-%, in einer Verpackung aus einem Material mit Barrierewirkung gegenüber Gas, die gasdicht verschlossen ist, enthalten ist.

2. Produkt nach Anspruch 1, **dadurch gekennzeichnet, dass** das Material der Verpackung einen Durchlässigkeitskoeffizienten für O₂ kleiner als 10 cm³/m²/24 Std aufweist.

3. Produkt nach einem der Ansprüche 1 oder 2, **dadurch gekennzeichnet, dass** das Material der Verpackung eine Barriere gegenüber Wasserdampf ist.

4. Produkt nach einem der vorstehenden Ansprüche, **dadurch gekennzeichnet, dass** das Material eine oder mehrere Schichten aus PVDC, aus OPA, aus EVOH oder aus Aluminium oder aus Gemischen davon umfasst.

5. Produkt nach einem der vorstehenden Ansprüche, **dadurch gekennzeichnet, dass** das Material Glas, Siliziumdioxid und/oder Aluminium umfasst.

6. Produkt nach einem der vorstehenden Ansprüche, **dadurch gekennzeichnet, dass** das Material UV-Schutz bietet.

7. Verfahren zur Herstellung eines Produkts nach einem der vorstehenden Ansprüche, **dadurch gekennzeichnet, dass** lebender Sauerteig oder Ähnliches in einen Behälter aus einem Material mit Barrierewirkung gegenüber Gasen unter Stickstoffatmosphäre, die zu mehr als 97 Vol.-% rein ist, gegeben wird, und der Behälter gasdicht verschlossen wird.

8. Verfahren nach Anspruch 7, **dadurch gekennzeichnet, dass** Stickstoff mit einer Reinheit zwischen 99 und 100 Vol.-% verwendet wird.

## Claims

1. A product consisting of leaven or the like, living, having a percentage of dry material by weight of less than 90%, **characterised in that** the product is contained in an atmosphere of nitrogen of purity greater than 97%, preferably between 99 and 100%, by volume in a package made of a gas barrier material and closed with a gas tight seal.

2. A product according to claim 1, **characterised in that** the packaging material has a permeability coefficient to O₂ of less than 10 cm³/m²/24 h.

3. A product according to claim 1 or 2, **characterised in that** the packaging material is a barrier to water vapour.

4. A product according to any one of the preceding claims, **characterised in that** the material consists of one or more layers of PVDC, OPA, EVOH or aluminum or mixtures thereof.

5. A product according to any one of the preceding claims, **characterised in that** the material consists of glass, silica and/or aluminum.

6. A product according to any one of the preceding claims, **characterised in that** the material is anti-UV.

7. A method of manufacturing a product according to any one of the preceding claims, **characterised in that** living leaven or the like is placed in a container made of a gas barrier material in an atmosphere of nitrogen of purity greater than 97% by volume and the container is closed with a gas tight seal.

8. A method according to claim 7, **characterised in that** nitrogen with a purity of between 99 and 100% by volume is used.
